# EUROPEAN PATENT APPLICATION

(11) **EP 0 818 212 A2**
(43) Date of publication of application: **14.01.1998**
(21) Application number: 97304891.1
(22) Date of filing: 04.07.1997
(51) Int. Cl.: A61M 16/00, G01N 33/00

(54) **Nitrogen dioxide monitors**

(30) Priority: 10.07.1996 US 21840 P; 13.12.1996 US 766834
(71) Applicant: OHMEDA INC., Liberty Corner, New Jersey 07938-0804 (US)
(72) Inventor: Bathe, Duncan P.L., Madison, Wisconsin 53711 (US); Montgomery, Frederick J., Sun Prairie, Wisconsin 53590 (US)
(74) Representative: Bousfield, Roger James

(57) **Abstract**

A method of correcting the readings of a NO₂ monitor in a system for administering a NO containing gas to a patient and oxygen through a supply conduit, the method comprising the steps of:
(a) withdrawing from the supply conduit a sample of the NO containing gas and oxygen being delivered a patient at a point in time,
(b) transferring the sample to the NO₂ monitor,
(c) determining the concentration of NO₂ in the sample by means of the NO₂ monitor and providing a reading indicative of such concentration,
(d) determining the concentration of O₂ and NO in the sample,
(e) determining the effective monitoring of time between the point of time that the sample is withdrawn in step (a) and the time the concentration of NO₂ is determined in the sample by the NO₂ monitor in step (c);
(f) calculating the amount of NO₂ generated in the sample conduit based on the time determined in step (e) and the concentration of O₂ and NO determined in step (d), and
(g) using the amount of NO₂ generated in the sample conduit as calculated in step (f) to correct the reading indicative of the concentration of NO₂ determined in step (c) to determine the concentration of NO₂ at the point the sample is withdrawn from the conduit.

## Description

This invention relates to a system for monitoring the concentration of nitrogen dioxide (NO₂) in a gas stream that is provided to a patient during the administration of nitric oxide (NO) and, more particularly, to a system for correcting the NO₂ measurements that are monitored by a gas sampling system.

Nitric oxide is generally administered to patient for various therapeutic reasons, among them, the treating or preventing of bronchoconstriction or reversible pulmonary vasoconstriction. One of such treatments is the administration of NO by means of inhalation and the treatment is more fully set forth in US Patent No. 5,485,827 of The General Hospital Corporation.

The administration of NO is accomplished by various types of apparatus, among them is the system disclosed in US Patent No. 5,558,083 in the present Applicant's name, the contents of which are incorporated herein by reference. In that system, an NO containing gas is provided as a gas in mixture of another inert gas, such as nitrogen, and the NO containing gas is mixed in a predetermined proportion with oxygen and administered to the patient.

One problem in the administration of NO with that or other methods, is that the NO reacts with oxygen to form NO₂ which is a toxic substance. The reaction of NO and O₂ to form NO₂ is time related, that is, the longer those components are admixed, the more NO₂ is formed in the mixture.

Obviously, therefore, it is very important that the concentration of NO₂ in the gases administered to the patient be carefully monitored to ensure that the level of NO₂ does not reach the toxic concentration to the patient. Therefore, a monitor must be used that continuously monitors the NO₂ level and equally important is that such a monitor provides the most accurate reading of the concentration of NO₂.

One of the types of monitors used for the detection of NO₂ is an electrochemical cell and which accurately detects the NO₂ concentration; however, the reading can be erroneous as an effect of the gas sampling system that takes a sample of the gas stream being administered to the patient and transmits that sample to the electrochemical monitor for analysis. As previously indicated, because the reaction of NO and O₂ is a time related reaction, the gas sampling system itself may introduce a source of error into the NO₂ detection system.

In the normal monitoring system, a side stream or sampling stream of gas is removed from the conduit carrying that gas stream to the patient and that side stream then conveys the sample to the NO₂ monitor. The difficulty arises in that the time between the actual removal of a sample of gas from the conduit to the patient and the actual analysis of the sample by the monitor allows the continuous reaction between NO and O₂ in that time period to increase the amount of NO₂ in the sample by the time the sample is actually analysed by the monitor.

Accordingly, the level of NO₂ that the monitor actually detects and indicates to the user is generally a higher amount than the actual concentration of NO₂ in the steam of gas being administered to the patient.

The present invention is concerned with the provision of a monitoring system which generally overcomes such difficulties.

The invention generally provides a system for correcting the reading of a NO₂ monitor to account for the elapsed time from when the sample of gas is removed from the stream of gas administered to the patient to when the monitor actually analyses the sample and provides a reading and/or signal representative of the NO₂ concentration.

In accordance with the invention, there is provided a method of correcting the readings of a NO₂ monitor in a system for administering a NO containing gas to a patient and oxygen through a supply conduit, the method comprising the steps of:
(a) withdrawing from the supply conduit a sample of the NO containing gas and oxygen being delivered a patient at a point in time,
(b) transferring the sample to the NO₂ monitor,
(c) determining the concentration of NO₂ in the sample by means of the NO₂ monitor and providing a reading indicative of such concentration,
(d) determining the concentration of O₂ and NO in the sample,
(e) determining the effective monitoring of time between the point of time that the sample is withdrawn in step (a) and the time the concentration of NO₂ is determined in the sample by the NO₂ is determined in the sample by the NO₂ monitor in step (c);
(f) calculating the amount of NO₂ generated in the sample conduit based on the time determined in step (e) and the concentration of O₂ and NO determined in step (d), and
(g) using the amount of NO₂ generated in the sample conduit as calculated in step (f) to correct the reading indicative of the concentration of NO₂ determined in step (c) to determine the concentration of NO₂ at the point the sample is withdrawn from the conduit.

In carrying out the invention, the method and associated system determines and takes into account the amount of time that passes between the obtaining of the sample at the sampling site and the actual analysis carried out by the monitor and uses that time to determine the additional amount of NO₂ that is formed by the reaction between NO and O₂ during that time period and thus corrects the reading of the monitor to provide a more accurate reading to the representative of the concentration of NO₂ in the stream of gas being administered to the patient.

For a better understanding of the invention, reference will be made by way of exemplification only, to the accompanying drawings in which:
FIG. 1 is a block diagram of a typical system for side stream monitoring of gases delivered to a patient; and
FIG. 2 is a block diagram of the steps utilised in carrying out the invention.

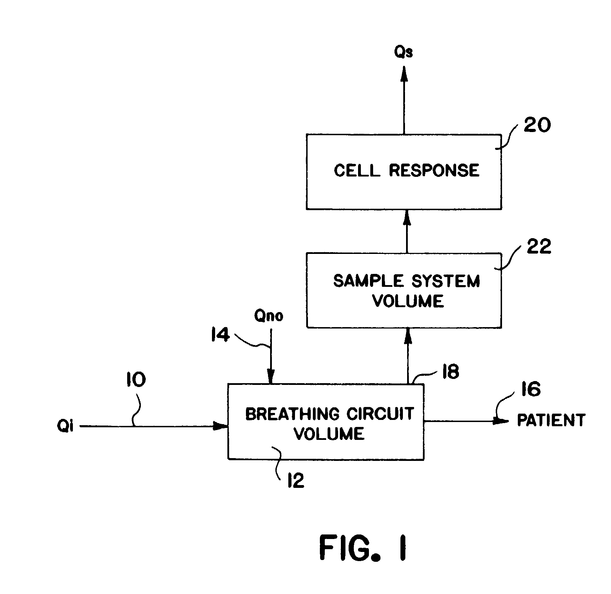

Turning to FIG. 1, a portion of a system for administering NO is shown and of complete system described in our US Patent No. 5,558,083. The stream of oxygen containing gas from the ventilator is depicted as a conduit 10 and which flow enters a breathing circuit 12. The flow of a gas containing NO is also administered to a breathing circuit 12 by means of a conduit 14. As seen in our US patent, a combined stream of an oxygen containing gas from the ventilator and the gas containing NO is combined and administered to a patient 16. As stated, the reaction between the oxygen contained in the gas entering in the conduit 10 and the NO entering in the conduit 14 causes a reaction resulting in the formation of NO₂ which is a toxic compound.

Accordingly, it is very important continuously to monitor the concentration of NO₂ in the patient breathing circuit 12 relatively close to the point that the gas stream is actually introduced into the patient 16. In FIG. 1, therefore, a sample point 18 is shown where a sample stream of the mixed NO containing gas is and O₂ containing gas is withdrawn from the breathing circuit and directed through a sample conduit to a monitor (not shown), generally of the electrochemical type, where the concentration of NO₂ in the sample gas is detected and a readout provided that may trigger an alarm system or otherwise provide notification to the user of the NO₂ concentration.

Since, however, the reaction of NO and O₂ to form NO₂ is a time related reaction, that reaction continues throughout the period from the point in time that the actual sample is taken at the sample point 18 to the point in time that the monitor actually determines the concentration of NO₂. That effective monitoring time (EMT) is determined by the flow and volume of the sample conduit and the electrochemical cell response time and is represented by the sample system volume in a block 22. The electrochemical cell response time is represented by a block 20.

Since NO₂ continues to be produced during that elapsed sampling time, the actual reading of the NO₂ concentration from the monitor is erroneous and will read high and not give the user a true, accurate reading of the NO₂ concentration of the NO₂ in the gas stream to the patient.

It is therefore necessary to determine in some manner, the effective monitoring time from the point that the sample is withdrawn from the breathing circuit 12 at sample point 18 to the time that the monitor actually analyses that sample to determine the NO₂ concentration. The effective monitoring time can be determined in a number of ways, one of which is to determine the flow in the sample conduit and to know or determine the volume in the sample conduit and the monitor response time. With those three values, the effective monitoring time of the sample between its removal from the conduit to the patient and the monitor can be readily determined.

It should be noted, however, that the NO₂ monitor may not be a sidestream monitor, that is, the NO₂ monitor may be directly receiving the sample gas from the conduit to the patient and therefore there is effectively no sample conduit and only the response time of the NO₂ monitor affects the production of NO₂. Thus, the effective monitoring time is, as will be explained, determined empirically to arrive at that time to use in the equation in determining NO₂generated in the reaction of NO and O₂.

As an alternate method of determining the effective monitoring time, it can be determined empirically by the system shown in FIG. 1 with the use of a fast NO₂ sensor such as the Binos Model 1004 ultra-violet absorbance spectrometer located directly at the sample point 18 and which provides a very rapid and accurate determination of the NO₂ concentration at that point.

The values are thus used in the following equation to determine the effective monitoring time:

Effective Monitoring Time = (ECMNO₂ - BinosNO₂)/{(**C**NO)²·**C**O₂.k}

Where:
ECMNO₂ is the measurement made of the NO₂ by electrochemical cell (ppm);
BinosNO₂ is the measurement of NO₂ by the Binos analyzer (ppm) at the sample point 18;
**C**NO is the concentration of NO (ppm) of the gas in the patient breathing circuit 12;
**C**O2 is the concentration of O₂ (ppm) of the gas in the patient breathing circuit 12;
k is a known constant; and

The Effective Monitoring Time (EMT) is in units of seconds.

The Binos monitor is accurate for the determination of NO₂ but its cost makes it prohibitive with the commercial NO administration equipment as described in the aforementioned patent. In any event, using the equation , k is a known constant and its value has been investigated and published by Sokol et al of the NICHD Neonatal Research Network, National Institute of Standards & Technology (NIST), Gaithersburg, MD and its value with the range of flows used with the NO administration equipment is about 1.27 × 10⁻¹¹ +/- 0.05 × 10⁻¹¹ Moles²/ppm²sec. and is therefor a known quantity in the equation.

Continuing on, a reading is taken for the NO₂ at the sampling point 18 with the Binos monitor and another reading is taken at the NO₂ monitor based on an electrochemical cell. Those readings are substituted into the prior equation to determine the sample effective monitoring time.

Thus a determination of the sample effective monitoring time can be calculated empirically for the particular system of sampling being used and that time used in a CPU to make the correction for NO₂ monitor being used.

Once the effective monitoring time has been determined for the particular system, the reading of the NO₂ concentration can be corrected by revising the equation to the following:

CNO₂sampling = k*(**C**NO)²***C**O₂*EMT

By this equation, the CPU simply solves for CNO₂sampling to obtain the value of NO₂ that is formed in the sample line during the time the sample is removed from the patient breathing circuit 12 at the sample point 18 to the time that the sample is actually analyzed by the NO₂ monitor and a value determined for the user. Once the value of that NO₂ produced due to sampling is obtained, the value is simply subtracted from the actual reading of the NO₂ monitor that is, the electrochemical sensor, to arrive at a value that is indicative of the NO₂ concentration at the sample point 18, that is, the NO₂ concentration of the gas stream introduced to the patient 16.

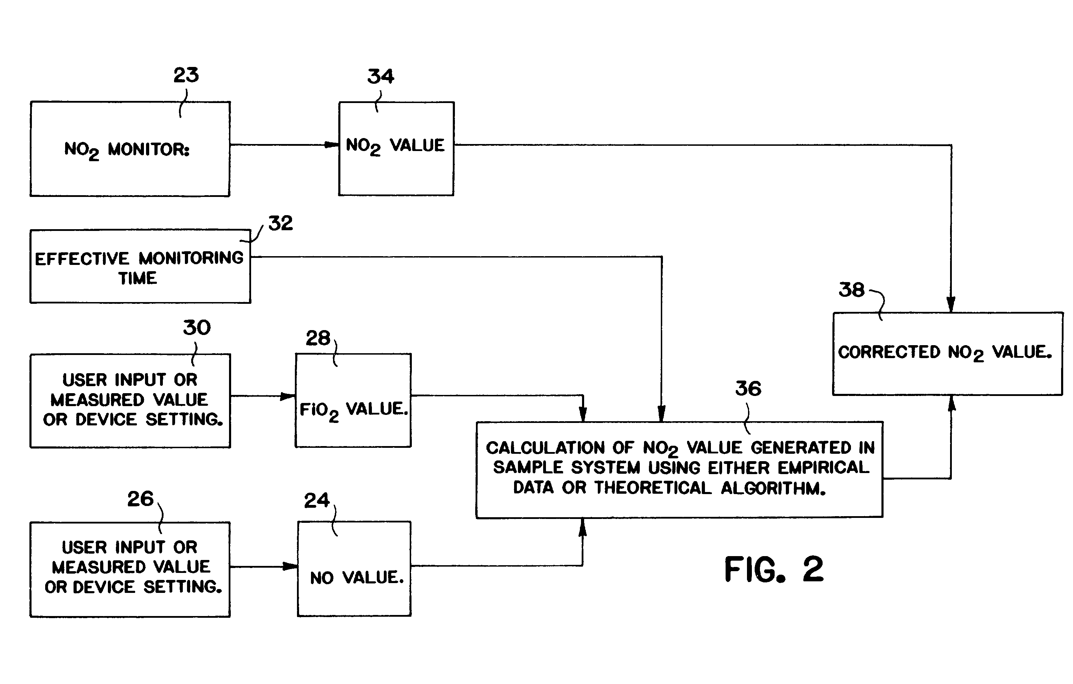

Turning now to FIG. 2, there is shown a block diagram of a system for correcting the readings from the NO₂ monitor 23 used in the FIG. 1 embodiment. As shown, a block 24 represents the value of NO that is being supplied to the patient from the overall NO administration system. The NO itself is provided by the NO supply. As noted, that supply is preferably a quantity of NO in mixture with nitrogen and typical concentrations in a gas cylinder may be in the range of 50 ppm to around 1000 ppm of NO in nitrogen. The actual value of that concentration of NO represented by the block 24 may be inputted from various means depicted by a block 26. Those means include a user input where a particular concentration of NO has been inputted by the user, a measured value from a NO monitor or may be a value set on the device that is providing the NO. In any event, that value of NO concentration of the gas supplied to the patient is known and represented by the block 24.

Along with that value, the value of the oxygen concentration in the stream of gas delivered to the patient, represented by a block 28, is used and, again, that value may be provided by a representative block 30 as a user input value, a measured value or may be a set value of the device itself.

Basically, therefore, the blocks 24 and 28 represent the concentrations of mixed gas containing both NO and O₂ and the mixture itself has been mixed in the NO gas administration apparatus in accordance with our aforementioned patent by combining a stream of NO containing gas and a stream of O₂ containing gas in a desired proportion to afford the proper and desired therapy to the patient.

As further input to the system, the sample system effective monitoring time that elapses from the point in time that the sample of the gas delivered to the patient is removed from the supply conduit to the patient and the point in time that the sample thus removed is actually analysed by the NO₂ monitor 23 and a reading provided. The effective monitoring time may be determined by a measurement or calculation of the volume in the sample circuit, the flow through that circuit and the monitor response time which also may be measured or calculated. Alternatively, the determination of the effective monitoring time may be derived through the empirical testing of the particular system by means of the equations previously referred to in this specification.

In any event, the effective measurement time represented by the block 32 is the time that the O₂ and NO are reacting in the sample line and is used by the CPU to determine the amount of NO₂ generated from that reaction during the sampling of the gas to the patient.

As a further data or value to the present monitor correction system, depicted in a block 34, the actual reading representative of the NO₂ concentration determined by the monitor having received the sample of gas from the patient circuit is used. Thus the value represented by the block 34 has had the further reaction of O₂ and NO that has taken place during the elapsed time the sample is removed from the patient breathing circuit to the time the monitor actually makes an analysis and provides a reading of the NO₂ concentration .

The aforementioned values of O₂ concentration, NO, and effective monitoring time are fed into a CPU at a block 36 where the data is processed with the aforedescribed equation to reach a value of the NO₂ created due to the reaction of NO and O₂ during the period of time that those constituents are together in the sample conduit. The result is a value of NO₂ and which is then used to correct the value of NO₂ detected by the NO₂ monitor 23 value of the block 34 by subtracting the calculated value from the detected value to arrive at a corrected NO₂ concentration. In conventional manner, that corrected value can then be used in a display to the user and/or used to trigger an alarm condition in the event of excess NO₂ concentration.

## Claims

1. A method of correcting the readings of a NO₂ monitor in a system for administering a NO containing gas to a patient and oxygen through a supply conduit, the method comprising the steps of:
(a) withdrawing from the supply conduit a sample of the NO containing gas and oxygen being delivered a patient at a point in time,
(b) transferring the sample to the NO₂ monitor,
(c) determining the concentration of NO₂ in the sample by means of the NO₂ monitor and providing a reading indicative of such concentration,
(d) determining the concentration of O₂ and NO in the sample,
(e) determining the effective monitoring of time between the point of time that the sample is withdrawn in step (a) and the time the concentration of NO₂ is determined in the sample by the NO₂ monitor in step (c);
(f) calculating the amount of NO₂ generated in the sample conduit based on the time determined in step (e) and the concentration of O₂ and NO determined in step (d), and
(g) using the amount of NO₂ generated in the sample conduit as calculated in step (f) to correct the reading indicative of the concentration of NO₂ determined in step (c) to determine the concentration of NO₂ at the point the sample is withdrawn from the conduit.

2. A method according to Claim 1 in which the step of determining the effective monitoring time comprises empirically testing of the sample conduit with a known sample of gas containing NO.

3. A method according to Claim 1 or Claim 2 in which the step of determining the effective monitoring time comprises calculating the time by using the flow through the sample conduit, the volume of the sample conduit and the response time of the NO₂ monitor.

4. A method according to any preceding claim in which the step of calculating the amount of NO₂ generated in the sample conduit is includes determining the O₂ and NO concentrations set by the user.

5. A method according to any preceding claim in which the step of calculating the amount of NO₂ generated in the sample conduit includes monitoring the values of NO and O₂ in the patient conduit.

6. A system for correcting the readings of a NO₂ monitor in a means for administering a NO containing gas and oxygen to a patient through a supply conduit, the system comprising:
a supply of a NO containing gas, a supply of a O₂ containing gas, means to mix said NO containing gas and the O₂ containing gas to a desired concentration of NO to supply the mixed gas to the patient through the supply conduit, means for removing a sample of the mixed gas from the supply conduit at a first point of time, an NO₂ monitor receiving the sample of mixed gas, said NO₂ monitor adapted to analyse the concentration of NO₂ in the sample of mixed gas and to provide a signal indicative of the concentration at a second point of time, means to determine the amount of NO₂ generated by the reaction of NO and O₂ during the period between the first and second points in time, and means to correct the signal indicative of the NO₂ concentration from said NO₂ monitor by using the amount of NO₂ generated to provide a corrected signal indicative of the NO₂ concentration of NO₂ in the gas being delivered to the patient.

7. A system according to Claim 6 in which the means to determine the amount of NO₂ generated comprises means empirically to determine the time between the first and second points in time.

8. A system according to Claim 6 or Claim 7 in which the means to determine the amount of NO₂ generated comprises sensing the concentrations of NO and O₂ in the sample of mixed gases.

9. A system according to any one of Claims 6 to 8 in which the means to determine the amount of NO₂ generated comprises a central processing unit (CPU).
